# EUROPEAN PATENT SPECIFICATION

(11) **EP 1 756 582 B1**
(45) Date of publication and mention of the grant of the patent: **12.01.2011**
(21) Application number: 05755429.7
(22) Date of filing: 09.06.2005
(51) Int. Cl.: G01N 33/68, G01N 33/569

(54) **MARKERS FOR ATHEROSCLEROSIS**
MARKER FÜR ATHEROSKLEROSE
MARQUEURS POUR ATHEROSCLEROSE

(30) Priority: 09.06.2004 GB 0412859
(43) Date of publication of application: 28.02.2007
(73) Proprietor: Immunoclin Ltd, London N12 8NP (GB)
(72) Inventor: BRAY, Dorothy, ImmunoClin Limited, London N12 8NP (GB); CLERICI, Mario, Milano University Medical School, I-20157 Milano (IT); TRABATTONI, Daria, Lucia, I-20033 Desio (IT)
(74) Representative: McQueen, Andrew Peter
(86) International application number: PCT/GB2005/002265
(87) International publication number: WO 2005/121804

(56) References cited:
- LEE W H ET AL: "Prevalence of foam cells and helper-T cells in atherosclerotic plaques of Korean patients with carotid atheroma." THE KOREAN JOURNAL OF INTERNAL MEDICINE. JUL 2000, vol. 15, no. 2, July 2000 (2000-07), pages 117-121, XP009053969 ISSN: 0494-4712
- RAMSHAW A L ET AL: "Combined immunohistochemical and immunofluorescence method to determine the phenotype of proliferating cell populations" JOURNAL OF CLINICAL PATHOLOGY (LONDON), vol. 45, no. 11, 1992, pages 1015-1017, XP009054051 ISSN: 0021-9746
- SANCHEZ-MARGALET V ET AL: "INFLAMMATORY RESPONSE TO CORONARY STENT IMPLANTATION IN PATIENTS WITH UNSTABLE ANGINA" CLINICAL CHEMISTRY AND LABORATORY MEDICINE, WALTER DE GRUYTER UND CO, DE, vol. 40, no. 8, August 2002 (2002-08), pages 769-774, XP009054038 ISSN: 1434-6621
- ERREN MICHAEL ET AL: "Systemic inflammatory parameters in patients with atherosclerosis of the coronary and peripheral arteries" ARTERIOSCLEROSIS THROMBOSIS AND VASCULAR BIOLOGY, vol. 19, no. 10, October 1999 (1999-10), pages 2355-2363, XP002346643 ISSN: 1079-5642

## Description

This invention relates to markers for atherosclerosis. More particularly the present invention relates to the identification of a number of markers found in the blood and to their use in aiding the diagnosis or prognosis of, or in identifying a predisposition towards atherosclerosis and especially the susceptibility to the development of atherosclerotic plaques and/or vascular obstruction.

Atherosclerosis is a pathological remodelling of the arteries which is a major cause of morbidity and mortality in developed countries; this pathology is the underlying basis of myocardial infarction, stroke and peripheral artery disease. Atherosclerosis can be considered as an unusual form of chronic inflammation occurring within the artery wall. A number of data support the pathogenic or pathogenetic role of inflammation in the aetiology of atherosclerosis; inflammation is an immunological phenomenon. One of the most convincing pieces of evidence for this statement stems from the observation that fatty streaks, the earliest detectable lesions in atherosclerosis, contain macrophage-derived foamy cells which are derived from circulating monocytes. T lymphocytes are also present in the atherosclerotic plaques and most of these are CD3+CD4+ alpha beta T cells. Such cells represent approximately 2/3 of all CD3+ T cells in advanced human lesions, and secrete IFN gamma, IL-2, TNF alpha and beta which cause vascular macrophage activation, vascular activation and inflammation. IFN gamma is thought to be responsible for plaque destabilization by reducing the fibrous cap. CD8+ T cells are also present in lesions but their contributory role in atherosclerosis is less clear.

The rolling, adhesion, and infiltration of immune cells (monocytes and CD4 T lymphocytes) which circulate in the peripheral blood within the endothelium is regulated by two major families of proteins expressed on the surface of immune cells, and on the surface of endothelial cells: these protein families are selectins and integrins. Chemokines or chemo-attractant cytokines are also extremely important in the control of cell trafficking and for the recruitment of immune cells at sites of inflammation. Some chemokines, those which can act as potent mediators of monocyte migration and macrophage differentiation, are expressed in human atherosclerotic lesions. In particular, macrophage chemoattractant protein 1 (MCP-1) and its specific receptor CCR2 are strongly believed to be involved in the initial stages of atherogenesis.

Additional support for the pathogenic or pathogenetic role of inflammation in the development of atherosclerotic plaques stems from the observation made by the present inventors that different cytokines preferentially trigger the development of atherosclerotic plaques (for example, TH1-like cytokines are "bad cytokines" within this biologic scenario, that is, they trigger or are involved in the triggering of the development of atherosclerotic plaques). This becomes clearer when it is considered that TH1 cytokines are prototypic inflammatory cytokines. Cytokines and other immunological proteins present in the atherosclerotic lesions are not only produced by CD4 T lymphocytes; in fact, fibroblast growth factors (FGFs) and platelet-derived growth factor (PDGF) are macrophage-derived growth factors which have been found to have significant effects on the pathology of atherosclerosis and which are macrophage-derived proteins. While the scientific knowledge of the composition of the atherosclerotic plaque and the contribution of cells found in lesions continues to increase, easy and reliable diagnosis of atherosclerosis, particularly in the absence of clinical symptoms remains a challenge as measuring and detecting changes in cell populations at the lesion site is not viable in clinical practice. Consequently there is a need for predictive and easy to assess markers that could be detected in peripheral blood samples.

The present inventors have found that the immunopathology of atherosclerotic plaques and alterations of the factors described above, and their specific receptors, result in changes of characteristics of multiple cell populations which can be measured in blood samples and in vascular biopsies of peripheral blood. Measures used may be: cell concentrations (percentages), expression of cell surface markers, intracellular concentrations of cytokines, chemokines or integrins, which alone or together with other parameters are useful in aiding the diagnosis or prognosis of, or in identifying a predisposition towards atherosclerosis and especially the susceptibility to the development of atherosclerotic plaques and/or vascular obstruction.

It is an object of the present invention to provide a method for the diagnosis, prognosis or identification of a predisposition towards atherosclerosis which eliminates or reduces the need for invasive techniques such as the removal of a plaque for identification or for the need to use invasive optical imaging techniques.

Accordingly, the present invention provides a method for the diagnosis, prognosis or identification of a predisposition towards atherosclerosis and the susceptibility to the development of atherosclerotic plaques and/or vascular obstruction, the method comprising analysing a sample to determine the percentage frequency of multiple sub-populations of T-lymphocytes and comparing the data obtained against comparative and/or control data, wherein at least one of said sub-populations is a sub-population of CD8+ T-lymphocytes selected from the sub-populations (phenotypes) of CD8+/CD11L+/CD45RA+, CD8+/CD11L +/CD45RO+, CD8+CD11 a+, CD8+CD62L+ and CD8+CD38+RO+, or a sub-population of CD4+ T lymphocytes selected from the following phenotypes: CD4+CD25+, CD4+DRII+, CD4+CD44+, CD4+CD62L+ and CD4+CD49d+.

### As used herein, "multiple" means at least two.

Preferably, the percentages or ratios of specific cell populations are determined.

### Conveniently, the sample is a blood or a biopsy sample.

In a preferred embodiment, the method comprises analysing a sample to determine the percentage frequency of one or more sub-populations of specific T-lymphocytes and comparing the data obtained against comparative and/or control data.

Preferably the method comprises determining the percentage frequency of one or more sub-populations of T-lymphocytes selected from the subpopulations (phenotypes) listed in Experiments 1 and 2 described herein.

In particular, the method comprises the determination of adhesion, activation, and rolling markers expressed on the surface of CD8+ T lymphocytes. Because these cells are the effector cells of the cellular arm of immunity, and because it is known that their activation can have a negative impact on disease development via destruction of target cells (e.g. the destruction of liver cell that are infected by hepatitis viruses is mediated by CD8 T cells and is associated with worsening of the disease) or via the induction of a chronic inflammatory condition, the inventors believe that focussing on these cells will allow a more accurate determination of the predisposition towards atherosclerosis and the susceptibility to the development of atherosclerotic plaques and/or vascular obstruction.

Additionally, the method comprises determining the simultaneous percentage frequency of multiple sub-populations of lymphocytes. This proposal stems from the fact that the predisposition towards atherosclerosis and the susceptibility to the development of atherosclerotic plaques and/or vascular obstruction is multi-factorial and thus it will is impossible to accurately predict such predisposition with the analysis of one single marker.

In a preferred embodiment, the method comprises determining the percentage frequency of multiple sub-populations of T-lymphocytes wherein at least one of said sub-populations is a sub-population of CD8+ T-lymphocytes and at least one other of said sub-populations is a sub-population of CD4+ T-lymphocytes. Preferably the CD8+ and CD4+ sub populations are selected from the phenotypes listed above.

Particularly preferred CD8+ sub-populations are: CD8+/CD11L+/CD45RA+ and CD8+/CD11L +/CD45RO+. Particularly preferred CD4+ sub-populations are CD4+CD62L+ and CD4+CD49d+. Ideally, one or more of each of the foregoing CD8+ and CD4+ sub-populations are measured together.

The biological reasons that led to the definition of such panel are detailed as follows:
- CD8/CD11L/CD45RA; post-naive cells that express CD11 (aka LFA-1alpha) with a low expression density, thus these are cells that express low levels of the adhesin CD11; this low expression makes them stick less to the endothelium. The reduction of these cells in atherosclerosis means that there are fewer circulating cells with a low "sticking ability" to the endothelium. These cells are similar to central memory cells and migrate to periphery as a likely effect of a 'negative feedback' resulting from plaque formation.
- CD8+/CD11L +/CD45RO+; these are CD8 T cells that are augmented in inflammation, similar to the phenotype of effector memory cells; these cells also have reduced ability of sticking to epithelium and may be a product of 'negative feedback' as a result of plaque formation.
- CD4/CD62L; CD62L is an adhesin (aka L selectin) thus an adhesion marker expressed on CD4 and utilized by these cells to adhere to the high endothelial venules (HERV) and penetrate within the vascular wall.
- CD4/CD25 and CD8/DR11; CD25 is the receptor for IL-2 and its expression is correlated with inflammation. DRII is expressed by CD4 and CD8 upon activation, thus the inventors' data confirms the inflammatory status associated with atherosclerosis.
- CD4/CD44 and CD4/CD49d; (cd44 aka M-CAM; CD49d aka VLA-4alpha); CD44 and CD49d are adhesion molecules; in theory they should increase in atherosclerosis; the decrease could be secondary to a negative feed-back (i.e. a way in which the body tries to stop the atherogenetic process).

The predictive value of the method of the invention may be augmented by supplementing the analysis of different lymphocyte sub-populations with further tests for other immunologic markers implicated in the development of atherosclerotic plaques and vascular obstruction. The use of multiple markers facilitates prediction of disease progression with better predictive value and, hence, provides a better outcome for the patient because appropriate treatment can be initiated earlier. Multiple immune correlates provide a powerful predictive tool because there is interdependence between the markers in contrast to routine diagnostics where very few results depend upon each other.

Accordingly, in a further embodiment, the invention provides a method for the diagnosis, prognosis or identification of a predisposition towards atherosclerosis and the susceptibility to the development of atherosclerotic plaques and/or vascular obstruction as described above, which further comprises determining the concentration of one or more soluble factors intracellularly, in plasma or in serum and comparing the data obtained against comparative and control data. Preferably, said soluble factor is selected from the group consisting of cytokines and chemokines. Most preferably, the cytokines are activation cytokines.

In a still further embodiment, the invention provides a method for the diagnosis, prognosis or identification of a predisposition towards atherosclerosis and the susceptibility to the development of atherosclerotic plaques and/or vascular obstruction as described above, which further comprises one or more of additional steps (a) and (b) :
(a) determining the percentage frequency of cells expressing activation cytokines, and/or chemokines, and/or integrins, and/or selectins in the sample;
(b) determining the frequency of cell surface molecules responsible for cell activation
and comparing the data obtained against comparative and control data.

It is preferred that the all of the above information is obtained from the same blood sample, and in addition, the chemokine and cytokine data may be obtained from serum or plasma. Additionally, the information may be obtained from a biopsy sample. However, it is preferred that the method is used with a blood sample in order to avoid or reduce the need for taking biopsy samples.

The preferred cytokines include interleukin-1 (α or β), interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interferon-α, interferon-β, interferon-γ, tumour necrosis factors such as TNF-α and TNF-β, fibroblast growth factors (FGF), platelet-derived growth factors (PDGF), Colony stimulating factors such as G-CSF, GM-CSF, M-LSF, and transforming growth factors such as TGF-β. Of these, TNF-α, IL-12, interferon-α, FGF, and PDGF are especially preferred.

### The preferred chemokines include CC and CXC chemokines.

The method may include the additional step of determination of the concentration of macrophage chemoattractant protein 1 (MCP-1) in a sample from the same patient.

Optionally, the method may also comprise the mathematical steps of determining the statistical changes in immunological parameters which may include lymphocyte population ratios; for example the ratio of CD8+/CD11L+/CD45RA+ lymphocytes to CD8+/CD11L+/CD45RO+ lymphocytes, as these two populations are likely to be a part of the lymphocyte maturation pathway and the ratios may therefore help to determine whether the maturation process is altered.

Embodiments of the invention will now be described purely by way of nonlimiting example in which reference is made to the figures of which:-
Figure 1 a to f. Describes changes in lymphocyte subpopulations in peripheral blood of HIV infected individuals who did (pl+) or did not (pl-) develop atherosclerotic plaques (carotid artery) as determined by echo doppler evaluations and in healthy controls.
Figure 2 a to h. Decribes changes in T cell populations in peripheral blood of HIV negative individuals who did (pl+) or did not (pl-) develop atherosclerotic plaques (carotid artery) as determined by echo doppler evaluations.

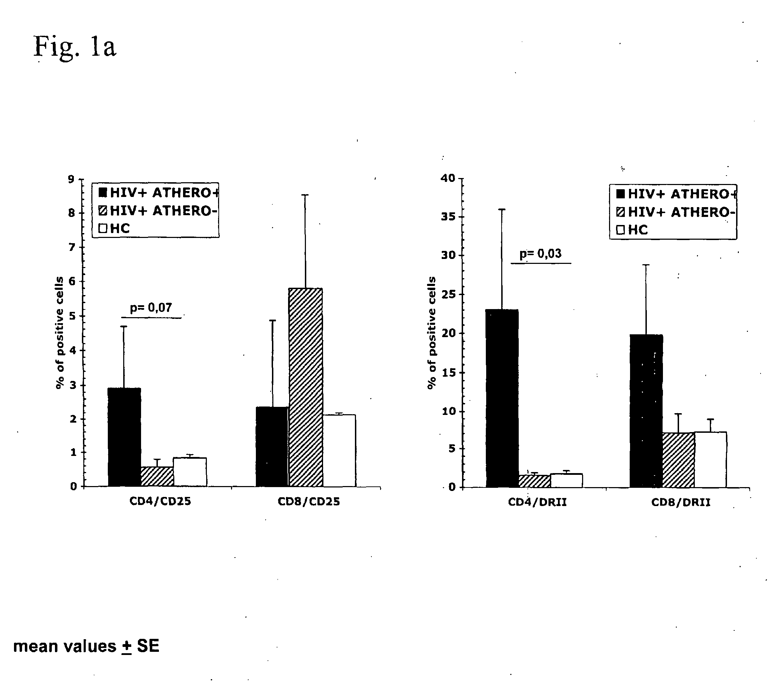

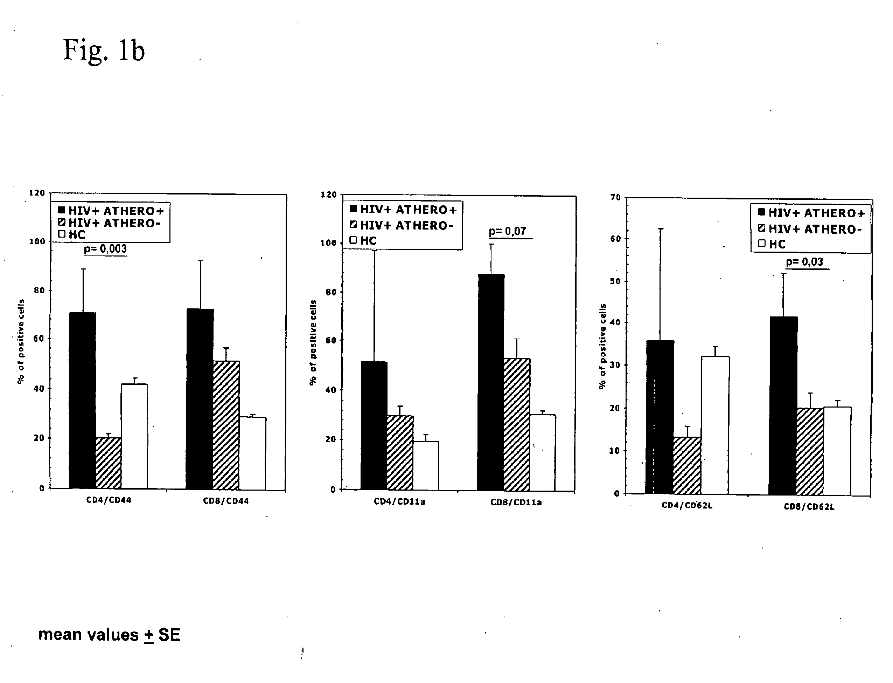

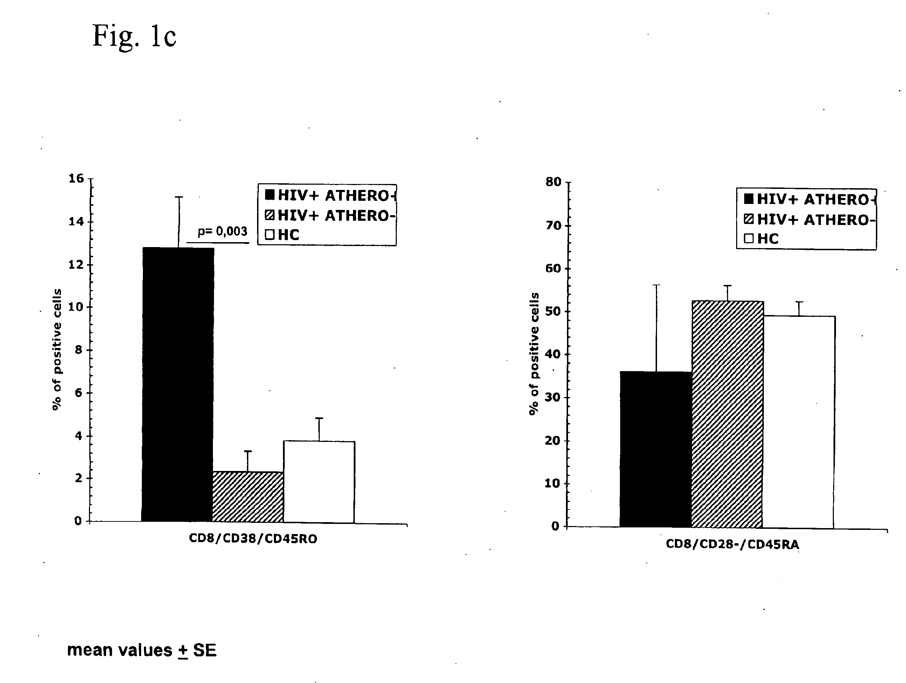

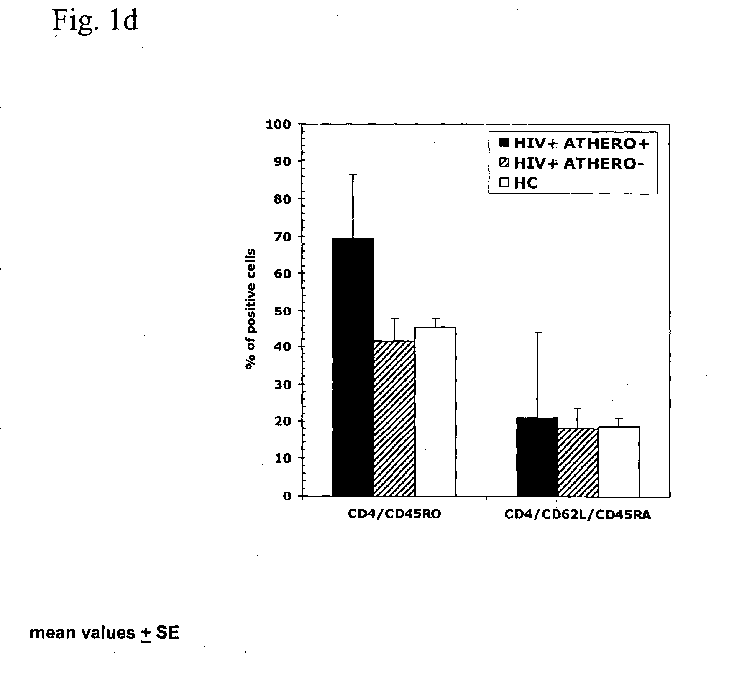

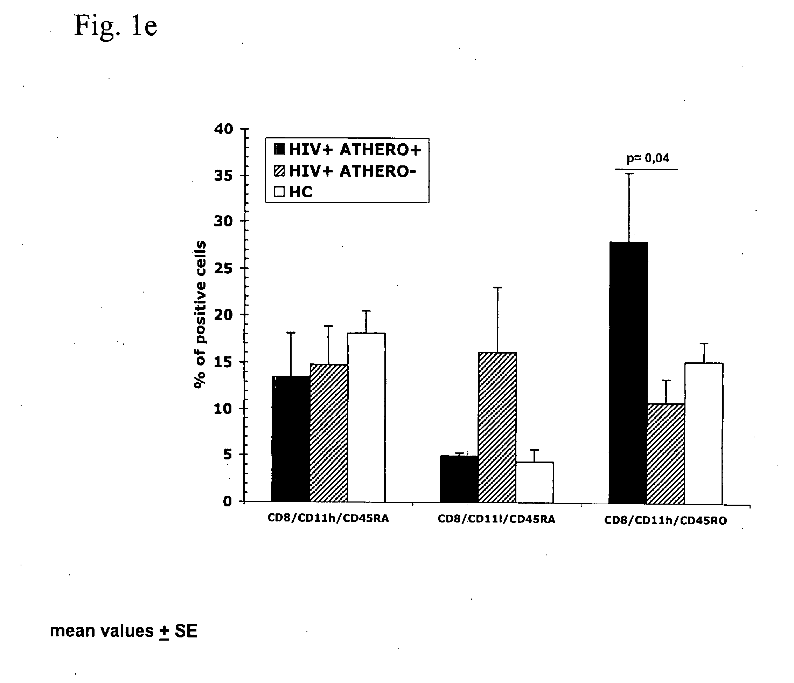

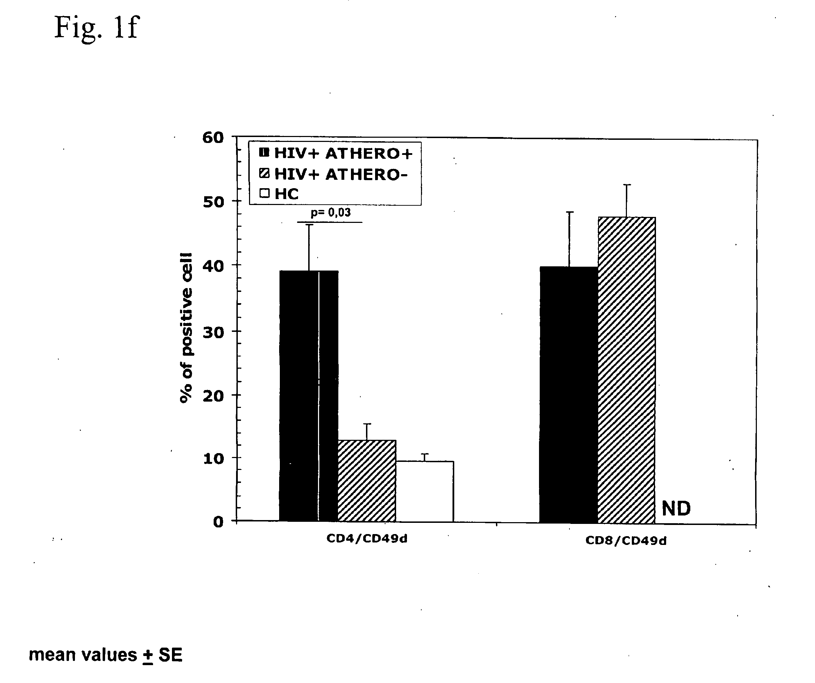

**Experiment 1:** T cell populations were assessed in peripheral blood of HIV infected individuals who did (pl+) or did not (pl-) develop atherosclerotic plaques (carotid artery) as determined by echo doppler evaluations. Data from 10 sex and age matched healthy controls were also obtained. The data are presented in Tables I and II and in Figure 1.

**Table I. Analysis of a number of different lymphocyte subpopulations by FACS methods in peripheral blood of HIV infected individuals who did (pl+) or did not (pl-) develop atherosclerotic plaques (carotid artery) as determined by echo doppler evaluations in Experiment 1.**

| **Phenotype** | **t-test** | **Mean** | |
|---|---|---|---|
| | **pl+ vs pl-** | **pl+** | **pl-** |
| **CD8+CD25+** | 0,374225892 | 2,36 | 5,80 |
| **CD4+CD25+** | **0,072409562** | 2,91 | 0,56 |
| **CD8+DRII+** | 0,282417879 | 19,83 | 7,11 |
| **CD4+DRII+** | **0,030600648** | 23,09 | 1,55 |
| **CD4+CD44+** | **0,00395716** | 70,60 | 20,36 |
| **CD8+CD44+** | 0,160297549 | 72,80 | 51,30 |
| **CD4+CD11a+** | 0,225448719 | 51,05 | 29,99 |
| **CD8+CD11a+** | **0,074474538** | 87,70 | 52,83 |
| **CD4+CD62L+** | 0,21745848 | 35,67 | 13,27 |
| **CD8+CD62L+** | **0,029642338** | 41,87 | 20,42 |
| **CD8+CD38+RO+** | **0,00295714** | 12,80 | 2,35 |
| **CD8+CD28-RA+** | 0,245463793 | 36,17 | 52,72 |
| **CD4+CD3+RO+** | **0,085958624** | 69,50 | 41,58 |
| **CD4+CD62L+RA+** | 0,654853858 | 20,98 | 18,12 |
| **CD8+CD11highRA+** | 0,166981489 | 13,57 | 14,80 |
| **CD8+CD11lowRA+** | 0,344892909 | 4,93 | 16,08 |
| **CD8+CD11highRO+** | **0,044961021** | 28,00 | 10,80 |
| **CD4+CD49d** | **0,034261111** | 39,10 | 12,98 |
| **CD8+CD49d** | ND | 40,10 | 47,80 |

The patients (N=15/group) were comparable in vitro-immunologic terms and had been undergoing a Pl-based ARV.

**Table II. Data obtained in 10 sex and age matched healthy controls in Experiment 1.**

| Phenotype | **Mean** | sd | **Se** |
|---|---|---|---|
| **CD8+CD25+** | **2,14** | 0,17591935 | **0,07** |
| **CD4+CD25+** | **0,84** | 0,22154221 | **0,09** |
| **CD8+DRII+** | **7,22** | 4,19724456 | **1,71** |
| **CD4+DRII+** | **1,77** | 0,9772532 | **0,4** |
| **CD4+CD44+** | **42,16** | 5,68561259 | **2,32** |
| **CD8+CD44+** | **29,21** | 3,33288092 | **1,36** |
| **CD4+CD11a+** | **19,60** | 5,92143001 | **2,42** |
| **CD8+CD11a+** | **30,93** | 3,40377941 | **1,39** |
| **CD4+CD62L+** | **32,19** | 5,790345 | **2,36** |
| **CD8+CD62L+** | **20,67** | 3,95462357 | **1,61** |
| **CD8+CD38+RO+** | **3,83** | 2,68271451 | **1,09** |
| **CD8+CD28-RA+** | **49,40** | 8,18005705 | **3,34** |
| **CD4+CD3+RO+** | **45,53** | 5,96984486 | **2,44** |
| **CD4+CD62L+RA+** | **18,64** | 5,45858869 | **2,23** |
| **CD8+CD11highRA+** | **18,10** | 5,76136558 | **2,35** |
| **CD8+CD11lowRA+** | **4,41** | 3,05692033 | **1,25** |
| **CD8+CD11highRO+** | **15,23** | 5,00723286 | **2,04** |
| **CD4+CD49d** | **9,57** | 2,21578802 | |
| **CD8+CD49d** | **NT** | | |

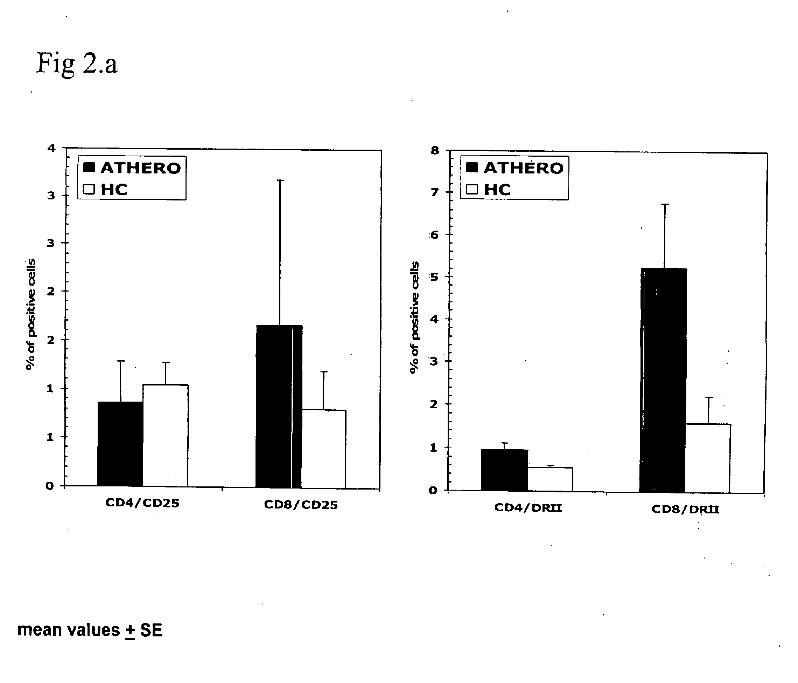

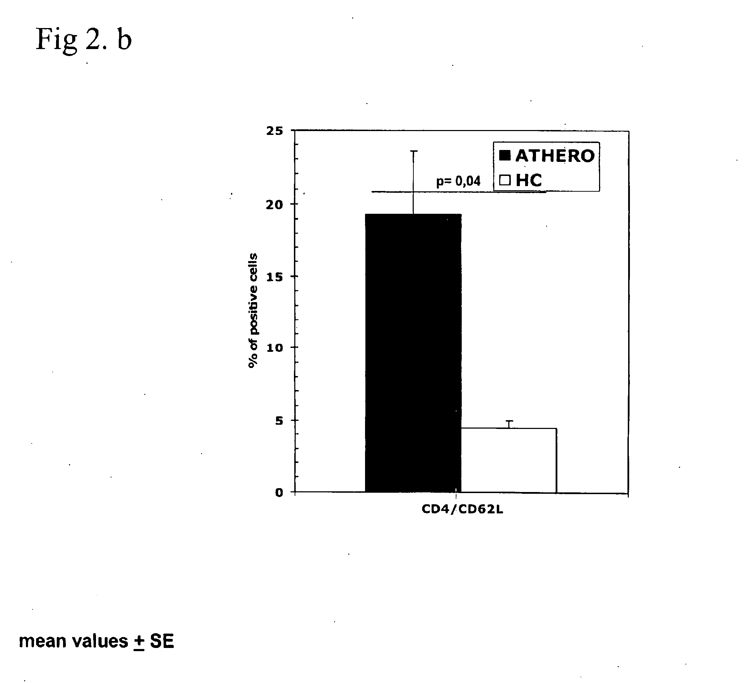

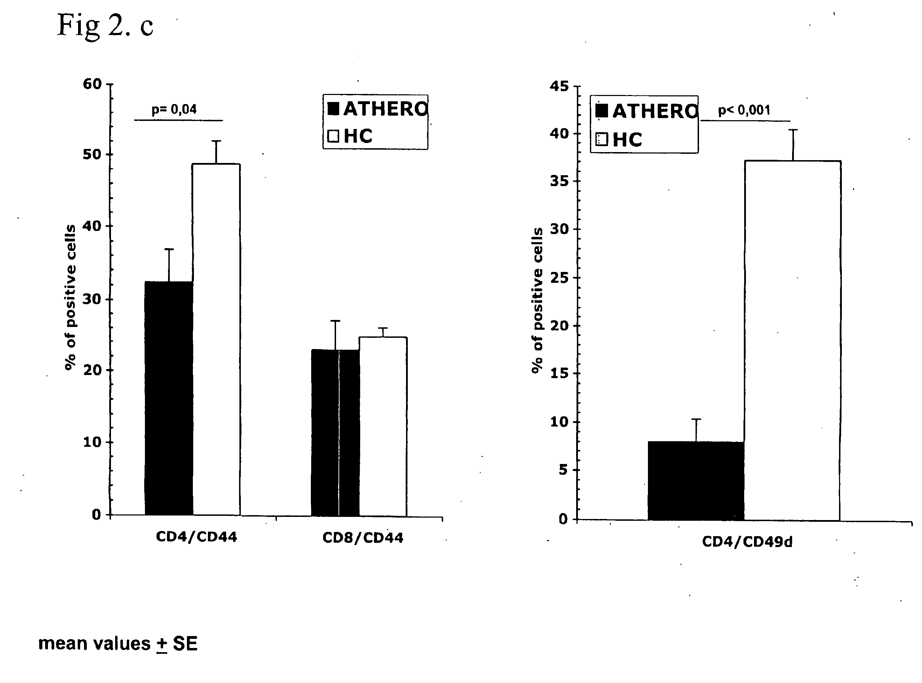

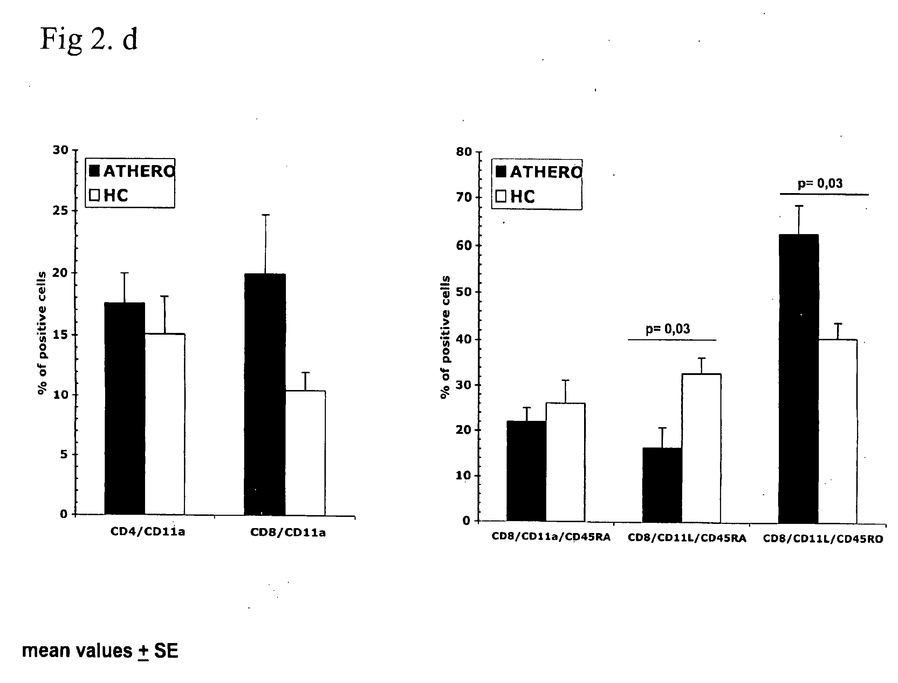

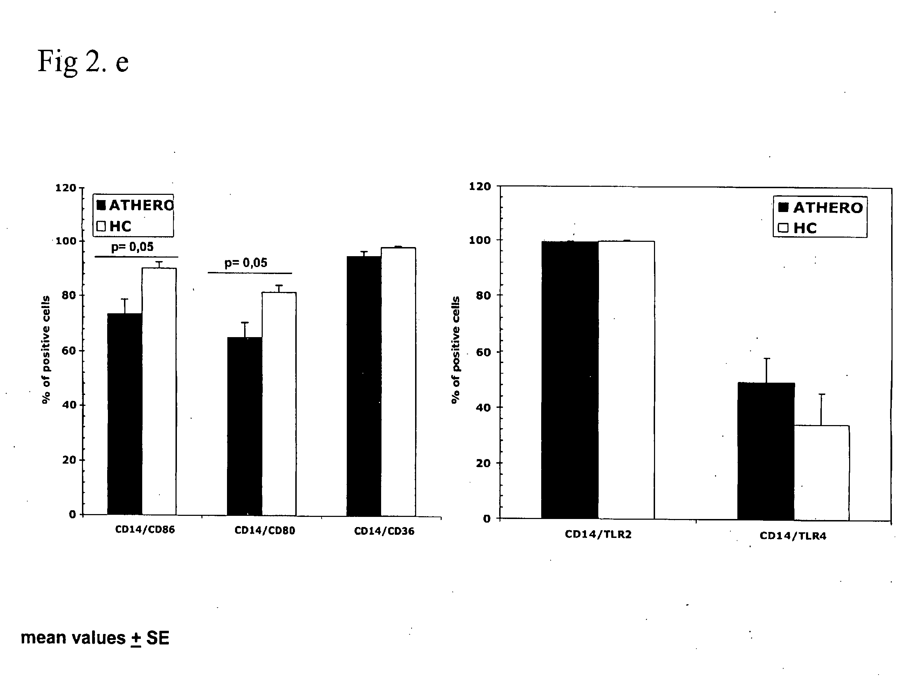

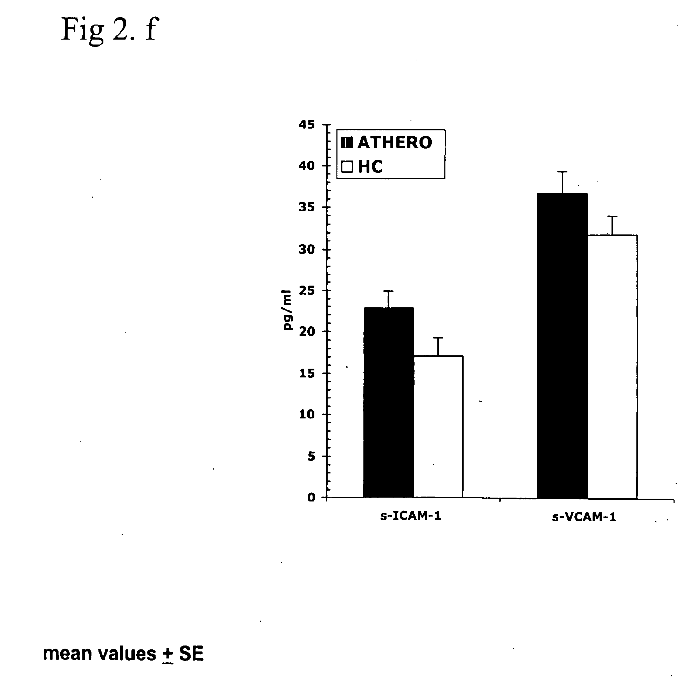

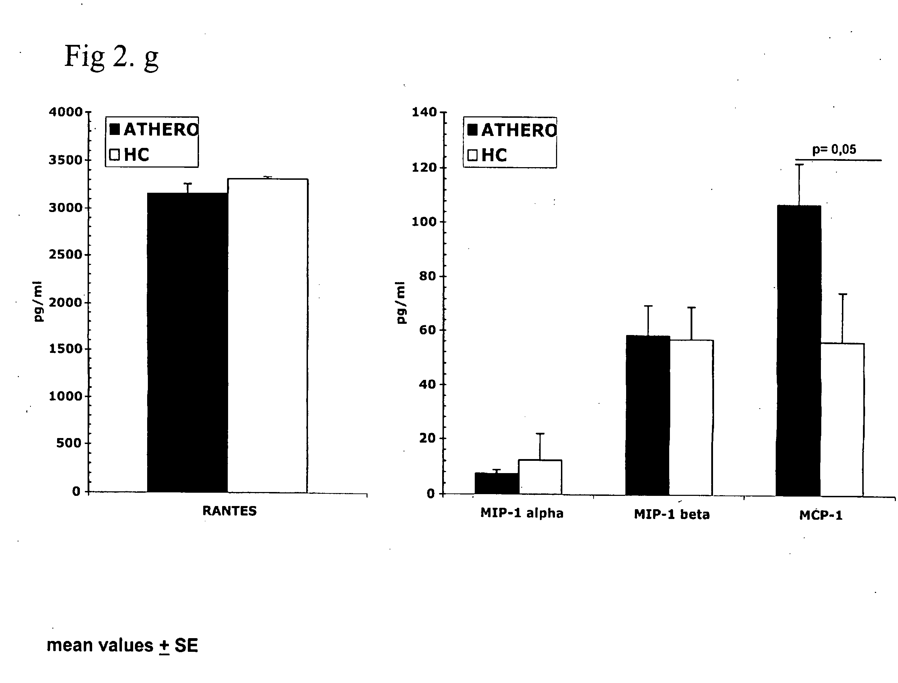

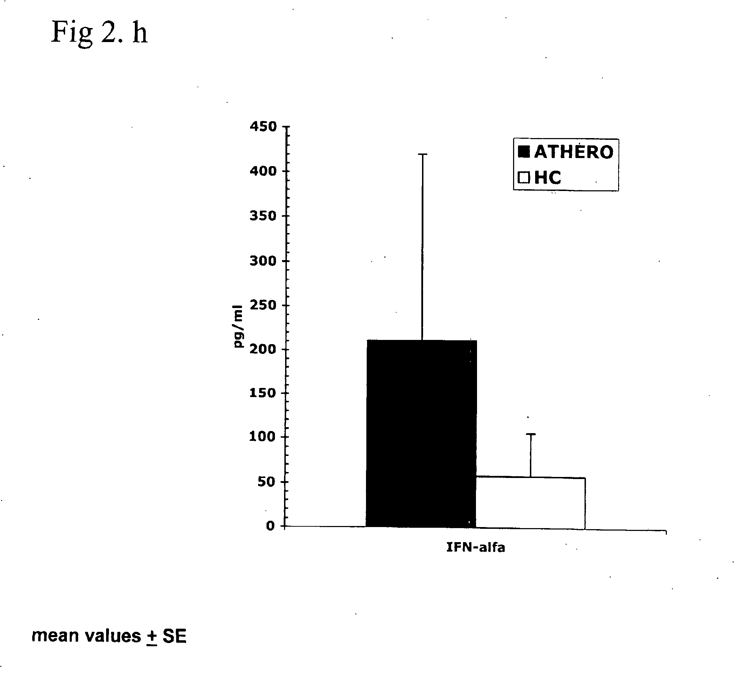

**Experiment 2:** T cell populations were assessed in peripheral blood of HIV negative individuals who did (pl+) or did not (pl-) develop atherosclerotic plaques (carotid artery) as determined by echo doppler evaluations. The data are presented in Table III and Figure 2.

The present inventors' initial observations were made on HIV infected patients. Because HIV is an immune deficiency, it is possible that the differences in lymphocyte populations observed in HIV infected patients may not be exactly mirrored in individuals no infected with HIV. Accordingly, the inventors studied lymphocyte populations in patients not having any other disease but with a positive diagnosis of thickening of the intima as determined by echo Doppler and in healthy controls as confirmed by echo Doppler.

When these data were compared with those for HIV infected patients, it was observed that several of the lymphocyte population markers were altered in the same direction (*i.e.* increased or decreased frequency in patients who developed plaques versus patients who did not) in the non-HIV infected patients as had previously been observed in the HIV infected patients. The HIV + patients in experiment 1 were in advanced stage of HIV disease and were treated with protease inhibitors which were to be associated with a statistically significant increase in the risk of cardiovascular disease. HIV disease is an immunodeficiency and dramatic alteration in lymphocyte markers is expected to be progressively greater with advanced disease. The uniformity of changes in multiple populations of lymphocytes in atherosclerotic patients both experiments is surprising in the view of the expected discrepancies between immunodeficient HIV infected population and non HIV infected patients. Accordingly, these data confirm the utility of changes in these lymphocyte populations as markers for atherosclerosis.

There were some discrepancies between the results of Experiment 1 (HIV infected patients) and Experiment 2 (non-HIV infected), but this was in lymphocyte populations known to be affected by HIV.

Lymphocyte populations which were observed to be altered in the same direction for patients who developed plaques in both the HIV infected and non-infected patient studies are the most promising diagnostic markers for atherosclerosis. However, it might be argued that lymphocyte populations which were observed to alter in opposite directions in the two experiments might still be useful as markers since an observed change of this population in a patient in either direction could be an indication that further investigation is warranted.

Interestingly, monocytic populations expressing CD86 and CD80 were found to be decreased in patients with atherosclerosis. These decreases may be a reflection of monocyte migration into atherosclerotic plaques.

Table III a and b shows phenotypic analysis of a number of different lymphocyte subpopulations in peripheral blood of individuals who developed atherosclerotic plaques (ATHERO1) and healthy controls (HC) in part a and levels of plasmatic cytokines in part b. It is of note that as expected levels of MCP-1 were higher in atherosclerosis patients as compared to healthy controls.

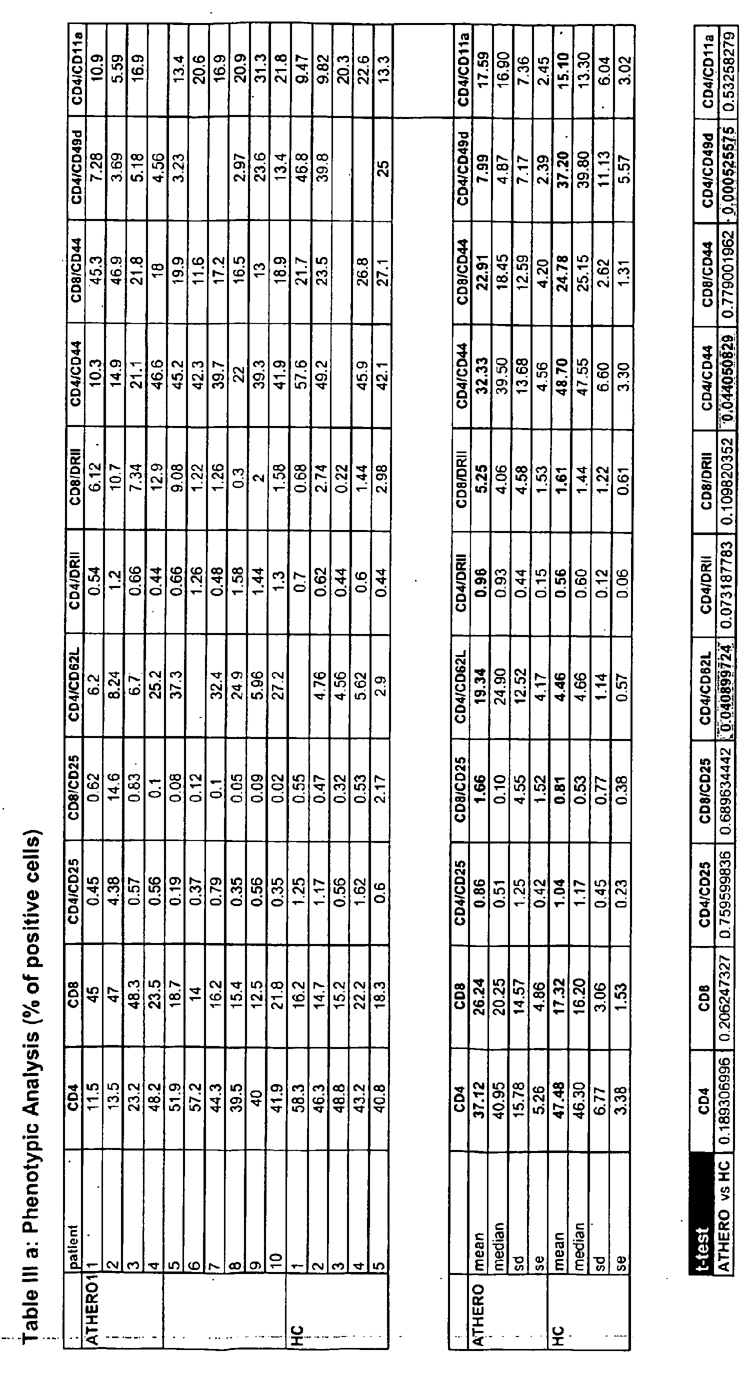

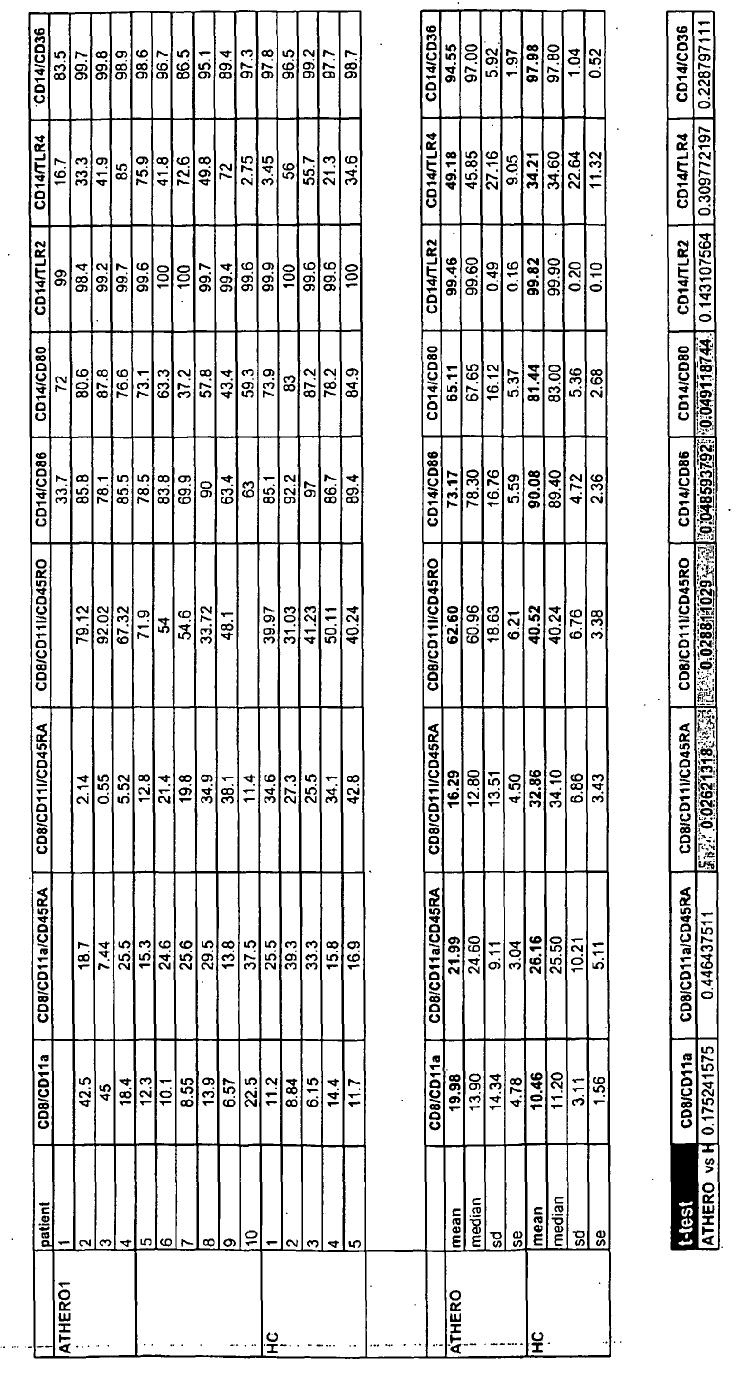

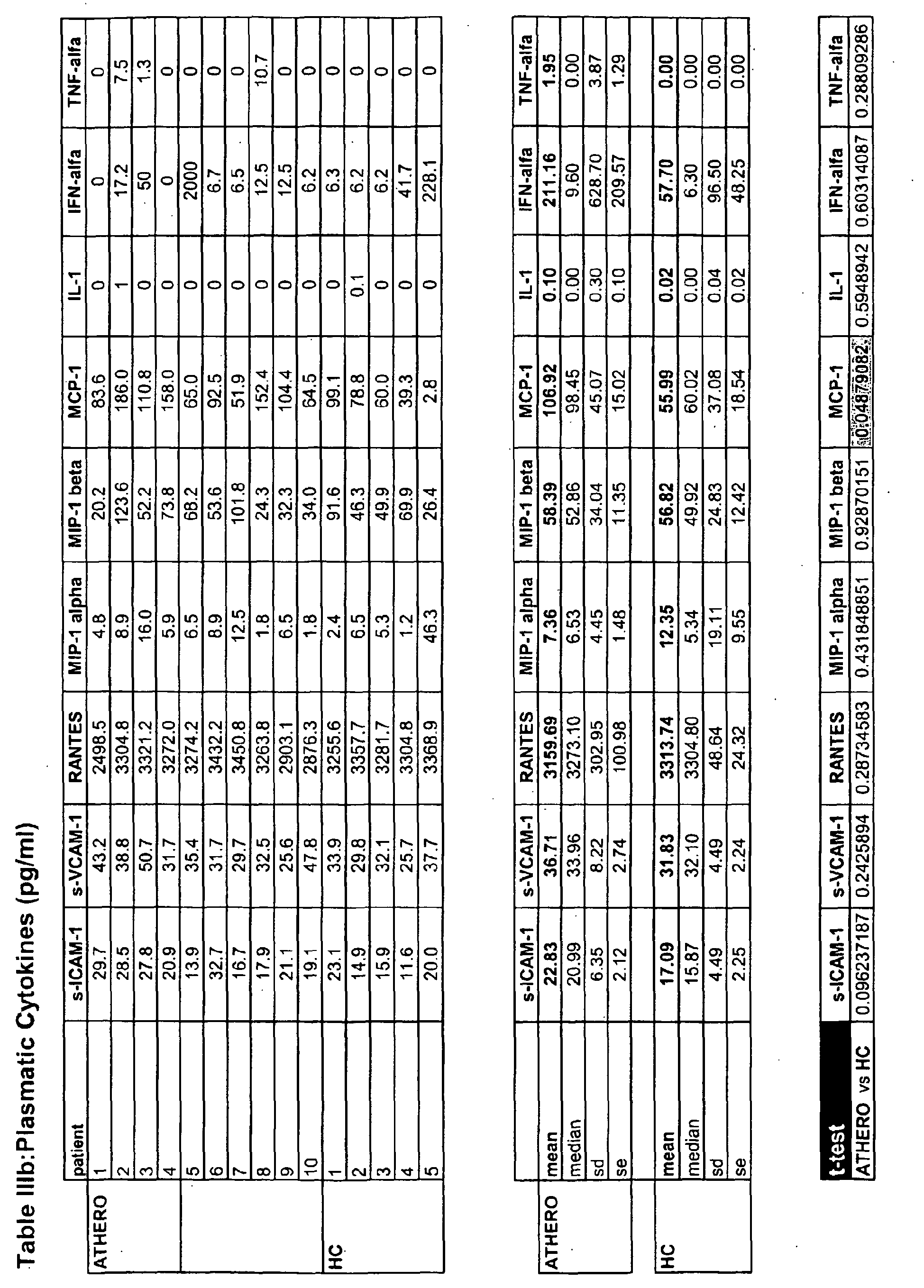

**Table IV. Lymphocyte markers cited in Experiments 1 and 2**

| **Marker** | **Description** | **Experiment 1 In HIV+ pts Pl+ vs. pl-** | **Experiment 2 In HIV neg pts Pl+ vs. PL-** |
|---|---|---|---|
| **CD8+CD25+** | CD25 is the receptor for IL-2 and its expression is correlated with inflammation. CD25-expressing lymphocytes are activated lymphocytes (an indirect index of inflammation) | Decrease in pl+ when compared to HIV+ but not when compared to healthy controls | Increase in pl+ |
| **CD4+CD25+** | CD25 is the receptor for IL-2 and its expression is correlated with inflammation. CD25-expressing lymphocytes are activated lymphocytes (an indirect index of inflammation) | Increase in pl+ | No change in Pl+ |
| **CD8+DRII+** | DRII is expressed by CD4 and CD8 upon activation. DRII-expressing lymphocytes: same value as CD25 | Increase in pl+ | Increase in pl+ |
| **CD4+DRII+** | DRII is expressed by CD4 and CD8 upon activation. DRII-expressing lymphocytes: same value as CD25 | Increase in pl+ | Increase in pl+ |
| **CD4+CD44+** | CD44 (M-CAM) is an adhesion molecule: circulating lymphocytes stick to the endothelial cells; this facilitates their penetration in the endothelium | Increase in pl+ | Decrease in Pl+ |
| **CD8+CD44+** | CD44 (M-CAM) is an adhesion molecule: circulating lymphocytes stick to the endothelial cells; this facilitates their penetration in the endothelium | Increase in pl+ | No change in pl+ |
| **CD4+CD11a+** | CD11 a is AlphaL integrin chain important for intercellular adhesions, and costimulatory signaling | Increase in pl+ | Increase in pl+ |
| **CD8+CD11a+** | CD11 a is AlphaL integrin chain important for intercellular adhesions, and costimulatory signaling | Increase in pl+ | Increase in pl+ |
| **CD4+CD62L+** | CD62L is an adhesin (L selectin). An adhesion marker expresed on CD4 and utilized by these cells to adhere to the high endothelial venules (HEV) and penetrate within the vascular wall. | Increase in pl+ | Increase in pl+ |
| **CD8+CD62L+** | CD62L is an adhesin (L selectin). An adhesion marker expresed on CD8 and utilized by these cells to adhere to the high endothelial venules (HEV) and penetrate within the vascular wall. | Increase in pl+ patients | Not done |
| **CD8+CD38+CD45RO+** | memory-activated T lymphocytes: an indirect index of inflammation and immune activation | Increase in pl+ patients | Not done |
| **CD8+CD28-CD45RA+** | T suppressor, IL-10-producing cells: the lower their value the worst the inflammatory condition | No change | Not done |
| **CD4+CD3+CD45RO+** | memory-activated T lymphocytes: an indirect index of inflammation and immune activation | Increase in pl+ | Not done |
| **CD4+CD62L+CD45RA+** | naive T lymphocytes | No change | Not done |
| **CD8+CD11 high+CD45RA+** | CD11 is an adhesion molecule: circulating lymphocytes stick to the endothelial cells; this facilitates their penetration in the endothelium | No change | Not done |
| **CD8+CD11low+CD45RA+** | CD11 is an adhesion molecule. Cells that express low levels of the adhesin CD11 makes them stick less to the endothelium.. | Decrease in Pl+ | Decrease in Pl+ |
| **CD8+CD11a+CD45RA+** | CD11 a is AlphaL integrin chain important for intercellular adhesions, and costimulatory signaling | Not done | No change |
| **CD8+CD11high+CD45RO+** | CD11 is an adhesion molecule: circulating lymphocytes stick to the endothelial cells; this facilitates their penetration in the endothelium | Increase in Pl+ patients | Not done |
| **CD8+CD11low+CD45RO+** | CD11 is an adhesion molecule; these are CD8 T cells that augment in inflammation. | Not done | Increase in pl+ |
| **CD4+CD49d+** | CD49 (VLA-4 alpha) is an adhesion molecule: circulating lymphocytes stick to the endothelial cells; this facilitates their penetration in the endothelium | Increase in Pl+ patients | Decrease in Pl+ |
| **CD8+CD49d+** | CD49 (VLA-a alpha) is an adhesion molecule: circulating lymphocytes stick to the endothelial cells; this facilitates their penetration in the endothelium | No change | Not done |
| **CD14+CD86+** | CD86 is also known as B7.2 Binds Cd28 responsible for co-stimulation of T cells, is thought to be mostly TH2 | Not done | Decrease in Pl+ |
| **CD14 +CD80+** | CD80 is also known CD80 also known as 87.1 ; it is thought to be mostly Th 1 | Not done | Decrease in Pl+ |
| **CD14 +TLR2+** | TLR2= TLR receptor | Not done | No change |
| **CD14 + TLR4+** | TL4=TLR receptor | Not done | No change |
| **CD14 + CD36+** | CD36 is activation marker | Not done | No change |

Preferably, the method includes the further step of assessing the thickness of the vascular intima to assess if there is thickening which may be indicative of the presence or likelihood of atherosclerosis or atherosclerotic plaques. In order to achieve this it is preferred that other non-invasive techniques such as ultrasonography, including Doppler ultrasound techniques, are used in conjunction with the method of the present invention. Further, the present invention may be used as a replacement for or in addition to conventional invasive imaging techniques.

The inventors have shown that alterations in the parameters described above, in particular, changes in T cell populations such as CD8+/CD11L+/CD45RA+, CD8+/CD11L +/CD45RO+, CD8+CD11a+, CD8+CD62L+, CD8+CD38+RO+, assessed together with CD4+CD3+RO+, CD8+CD11highRO+, CD4+CD62L+, CD4+CD49d+, CD4+CD25+, CD4+DRII+, CD4+CD44+, are indicative of plaque formation. Where analysis according to the invention indicates a high risk that a plaque has formed or is imminent, this may be confirmed by invasive test. Advantageously, the test according to the invention is performed first at regular intervals and the invasive test only performed if the raised frequency for one or more of the indicator cell populations is observed.

It is envisaged that data from the method of the invention will be interpreted using computer software and graphic representation.

Methods used to measure cells include: labelling of whole blood in a single tube; lysing red blood cells; and reading using flow cytometry. Alternatively, labelled cells may be added to a solid phase whereby cells are counted using an image analyzer and customized software is used to provide automated output of data: output may be tailored to compare single patients' results to a database of results from healthy individuals and to a database of data from varying stages of the disease to facilitate accurate prediction of plaque formation.

## Claims

1. A method for the diagnosis, prognosis or identification of a predisposition towards atherosclerosis and the susceptibility to the development of atherosclerotic plaques and/or vascular obstruction, the method comprising analysing a sample to determine the percentage frequency of at least two sub-populations of T-lymphocytes and comparing the data against comparative and/or control data, wherein at least one of said subpopulations of T-lymphocytes is a CD8 T cell population selected from the list consisting of CD8+/CD11L+/CD45RA+, CD8+/CD11L+/CD45RO+, CD8+CD11 a+, CD8+CD62L+, CD8+CD11 highRO+ and CD8+CD38+RO+, or a CD4 T cell population selected from the list consisting of CD4+/62L+, CD4+CD49d+, CD4+CD25+, CD4+DRII+, CD4+CD44+ and CD4+CD3+RO+.

2. A method according to claim 1, wherein at least one of said subpopulations of T-lymphocytes is a CD8 T cell population selected from the list consisting of CD8+/CD11L+/CD45RA+, CD8+/CD11L+/CD45RO+, CD8+CD11 a+, CD8+CD62L+, CD8+CD11 highRO+ and CD8+CD38+RO+ and at least one other of said sub-populations of T-lymphocytes is a CD4 T cell population selected from the list consisting of CD4+/62L+, CD4+CD49d, CD4+CD25+, CD4+DRII+, CD4+CD44+ and CD4+CD3+RO+

3. A method according to claim 1 or claim 2, wherein the sample is a blood or a biopsy sample.

4. A method according to any preceding claim which further comprises determining the concentration of one or more soluble factors intracellularly, in plasma or in serum and comparing the data obtained against comparative and control data.

5. A method according to claim 4, wherein said soluble factor is selected from the group consisting of cytokines and chemokines.

6. A method according to any preceding claim which further comprises one or more of additional steps (a) and (b) :
(a) determining the percentage frequency of cells expressing activation cytokines, and/or chemokines, and/or integrins, and/or selectins in the sample;
(b) determining the frequency of cell surface molecules responsible for cell activation
and comparing the data obtained against comparative and control data.

7. A method according to claim 5 or 6, in which the cytokine is one or more of interleukin-1 (α or β), interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interferon-α, interferon-β, interferon-γ, tumour necrosis factors such as TNF-α and TNF-(β, fibroblast growth factors (FGF), platelet-derived growth factors (PDGF), Colony stimulating factors such as G-CSF, GM-CSF, M-LSF, transforming growth factors such as TGF-β.

8. A method according to claim 7, in which the cytokines are TNF-α, IL-12, interferon-α, FGF, and PDGF.

9. A method according to claim 5 or 6, in which the chemokines include CC and CXC chemokines.

10. A method according to any preceding claim, in which the method further comprises the additional step of determination of the concentration of macrophage chemoattractant protein 1 (MCP-1) in a sample from the same patient.

11. A method according to any preceding claim, in which the method further comprises the additional step of determining the statistical changes in immunological parameters.

12. A method according to claim 11, in which the immunological parameter is the lymphocyte ratios.

13. A method according to any preceding claim, in which the method further comprises the step of assessing the thickness of the vascular intima to determine if there is thickening which may be indicative of the presence or likelihood of atherosclerosis or atherosclerotic plaques.

14. A method according to claim 13, in which the assessment of the thickness of the vascular intima is by ultrasonography.

15. A method according to any preceding claim, in which the data is interpreted using computer software and graphic imaging.

16. A method according to claim 3, in which analysis of the blood sample comprises labelling of whole blood in a single tube.

17. A method according to claim 16, in which analysis of the blood sample comprises the further steps of lysing red blood cells, and reading using flow cytometry.

18. A method according to claim 16, in which analysis of the blood sample comprises the further steps of adding the labelled cells to a solid phase, and using an image analyzer to count cells.

19. A method according to claim 17 or 18, in which computer software is used to compare single patients' results to a database of healthy individuals' results and to database of data from varying stages of the disease to facilitate accurate prediction of the plaque formation.

## Patentansprüche

1. Verfahren zur Diagnose, Prognose oder Identifizierung einer Veranlagung für Atherosklerose und der Empfänglichkeit für die Entwicklung von atherosklerotischen Plaques und/oder Gefäßobstruktion, wobei das Verfahren das Analysieren einer Probe, um die prozentuale Häufigkeit von mindestens zwei Subpopulationen von T-Lymphozyten zu bestimmen, und das Vergleichen der Daten mit Vergleichsund/oder Kontrolldaten umfasst, wobei mindestens eine der Subpopulationen von T-Lymphozyten eine CD8-T-Zellpopulation, die aus der Liste bestehend aus CD8+/CD11L+/CD45RA+, CD8+/CD11L+/CD45RO+, CD8+CD11a+, CD8+CD62L+, CD8+CD11highRO+ und CD8+CD38+RO+ ausgewählt ist, oder eine CD4-T-Zellpopulation ist, die aus der Liste bestehend aus CD4+/62L+, CD4+CD49d+, CD4+CD25+, CD4+DRII+, CD4+CD44+ und CD4+CD3+RO+ ausgewählt ist.

2. Verfahren nach Anspruch 1, wobei mindestens eine der Subpopulationen von T-Lymphozyten eine CD8-T-Zellpopulation ist, die aus der Liste bestehend aus CD8+/CD11L+/CD45RA+, CD8+/CD11L+/CD45RO+, CD8+CD11a+, CD8+CD62L+, CD8+CD11highRO+ und CD8+CD38+RO+ ausgewählt ist, und mindestens eine der Subpopulationen von T-Lymphozyten eine CD4-T-Zellpopulation ist, die aus der Liste bestehend aus CD4+/62L+, CD4+CD49d, CD4+CD25+, CD4+DRII+, CD4+CD44+ und CD4+CD3+RO+ ausgewählt ist.

3. Verfahren nach Anspruch 1 oder 2, wobei es sich bei der Probe um eine Blutprobe oder eine Biopsieprobe handelt.

4. Verfahren nach einem der vorhergehenden Ansprüche, das weiterhin das Bestimmen der Konzentration eines oder mehrerer löslicher Faktoren intrazellulär, in Plasma oder in Serum und das Vergleichen der erhaltenen Daten mit Vergleichs- und Kontrolldaten umfasst.

5. Verfahren nach Anspruch 4, wobei der lösliche Faktor aus der Gruppe bestehend aus Zytokinen und Chemokinen ausgewählt ist.

6. Verfahren nach einem der vorhergehenden Ansprüche, das weiterhin einen oder mehrere zusätzliche Schritte (a) und (b) umfasst:
(a) Bestimmen der prozentualen Häufigkeit von Zellen, die Aktivierungszytokine und/oder Chemokine und/oder Integrine und/oder Selektine exprimieren, in der Probe;
(b) Bestimmen der Häufigkeit von Zelloberflächenmolekülen, die für die Zellaktivierung verantwortlich sind,
und Vergleichen der erhaltenen Daten mit Vergleichs- und Kontrolldaten.

7. Verfahren nach Anspruch 5 oder 6, wobei es sich bei dem Zytokin um eines oder mehrere von Interleukin-1 (α oder β), Interleukin-2, Interleukin-3, Interleukin-4, Interleukin-5, Interleukin-6, Interleukin-7, Interleukin-8, Interleukin-9, Interleukin-10, Interleukin-11, Interleukin-12, Interleukin-13, Interleukin-14, Interleukin-15, Interleukin-16, Interleukin-17, Interferon-α, Interferon-β, Interferon-γ, Tumor-Nekrose-Faktoren wie TNF-α und TNF-β, Fibroblastenwachstumsfaktoren (FGF), aus Blutplättchen gewonnenen Wachstumsfaktoren (PDGF), koloniestimulierenden Faktoren wie G-CSF, GM-CSF, M-LSF, von transformierten Zellen gebildeten Wachstumsfaktoren wie TGF-β handelt.

8. Verfahren nach Anspruch 7, wobei es sich bei den Zytokinen um TNF-α, IL-12, Interferon-α, FGF und PDGF handelt.

9. Verfahren nach Anspruch 5 oder 6, wobei die Chemokine CC- und CXC-Chemokine beinhalten.

10. Verfahren nach einem der vorhergehenden Ansprüche, wobei das Verfahren weiterhin den zusätzlichen Schritt der Bestimmung der Konzentration von Makrophagen-chemoattraktivem Protein 1 (MCP-1) in einer Probe von demselben Patienten umfasst.

11. Verfahren nach einem der vorhergehenden Ansprüche, wobei das Verfahren weiterhin den zusätzlichen Schritt des Bestimmens der statistischen Veränderungen immunologischer Parameter umfasst.

12. Verfahren nach Anspruch 11, wobei es sich bei dem immunologischen Parameter um die Lymphozyten-Anteile handelt.

13. Verfahren nach einem der vorhergehenden Ansprüche, wobei das Verfahren weiterhin den Schritt des Beurteilens der Dicke der Gefäßintima umfasst, um zu bestimmen, ob eine Verdickung vorliegt, die das Vorliegen oder die Wahrscheinlichkeit von Atherosklerose oder atherosklerotischen Plaques anzeigen kann.

14. Verfahren nach Anspruch 13, wobei die Beurteilung der Dicke der Gefäßintima mittels Ultraschalldiagnostik erfolgt.

15. Verfahren nach einem der vorhergehenden Ansprüche, wobei die Daten unter Verwendung von Computersoftware und graphischer Abbildung ausgewertet werden.

16. Verfahren nach Anspruch 3, wobei die Analyse der Blutprobe die Markierung von Vollblut in einem einzigen Reagenzglas umfasst.

17. Verfahren nach Anspruch 16, wobei die Analyse der Blutprobe die weiteren Schritte des Lysierens roter Blutzellen und des Lesens unter Verwendung von Durchflusszytometrie umfasst.

18. Verfahren nach Anspruch 16, wobei die Analyse der Blutprobe die weiteren Schritte des Zugebens der markierten Zellen zu einer festen Phase und des Verwendens eines Bildanalysegeräts zum Zählen der Zellen umfasst.

19. Verfahren nach Anspruch 17 oder 18, wobei Computersoftware verwendet wird, um die Ergebnisse von Einzelpatienten mit einer Datenbank von Ergebnissen von gesunden Patienten und mit einer Datenbank von Daten von verschiedenen Stadien der Erkrankung zu vergleichen, um eine präzise Vorhersage der Plaquebildung zu erleichtern.

## Revendications

1. Procédé pour le diagnostic, le pronostic ou l'identification d'une prédisposition pour l'athérosclérose et la susceptibilité au développement de plaques d'athérosclérose et/ou d'une obstruction vasculaire, le procédé comprenant l'analyse d'un échantillon pour déterminer la fréquence en pourcentage d'au moins deux sous-populations de lymphocytes T et la comparaison des données par rapport à des données comparatives et/ou témoins, dans lequel au moins une desdites sous-populations de lymphocytes T est une population de cellules T CD8 sélectionnée parmi la liste constituée de CD8+/CD11L+/CD45RA+, CD8+/CD11L+/CD45RO+, CD8+CD11a+, CD8+CD62L+, CD8+CD11highRO+ et CD8+CD38+RO+, ou une population de cellules T CD4 sélectionnée parmi la liste constitué de CD4+/62L+, CD4+CD49d+, CD4+CD25+, CD4+DRII+, CD4+CD44+ et CD4+CD3+RO+.

2. Procédé selon la revendication 1, dans lequel au moins une desdites sous-populations de lymphocytes T est une population de cellules T CD8 sélectionnée parmi la liste constituée de CD8+/CD11L+/CD45RA+, CD8+/CD11L+/CD45RC+, CD8+CD11a+, CD8+CD62L+, CD8+CD11highRO+ et CD8+CD38+RO+ et au moins une autre desdites sous-populations de lymphocytes T est une population de cellules T CD4 sélectionnée parmi la liste constituée de CD4+/62L+, CD4+CD49d, CD4+CD25+, CD4+DRII+, CD4+CD44+ et CD4+CD3+RO+.

3. Procédé selon la revendication 1 ou la revendication 2, dans lequel l'échantillon est un échantillon de sang ou de biopsie.

4. Procédé selon l'une quelconque des revendications précédentes, qui comprend en outre la détermination de la concentration d'un ou de plusieurs facteurs solubles de manière intracellulaire, dans du plasma ou dans du sérum et la comparaison des données obtenues par rapport aux données comparatives ou témoins.

5. Procédé selon la revendication 4, dans lequel ledit facteur soluble est sélectionné parmi le groupe constitué de cytokines et de chimiokines.

6. Procédé selon l'une quelconque des revendications précédentes qui comprend en outre une ou plusieurs étapes parmi les étapes (a) et (b) supplémentaires :
(a) détermination de la fréquence en pourcentage de cellules exprimant des cytokines, et/ou chimiokines, et/ou intégrines, et/ou sélectines d'activation dans l'échantillon ;
(b) détermination de la fréquence des molécules de surface cellulaire responsables de l'activation cellulaire
et comparaison des données obtenues par rapport aux données comparatives et témoins.

7. Procédé selon la revendication 5 ou la revendication 6, dans lequel la cytokine est une ou plusieurs parmi interleukine-1 (α ou β), interleukine-2, interleukine-3, interleukine-4, interleukine-5, interleukine-6, interleukine-7, interleukine-8, interleukine-9, interleukine-10, interleukine-11, interleukine-12, interleukine-13, interleukine-14, interleukine-15, interleukine-16, interleukine-17, interféron-α, interféron-β, interféron-γ, facteurs de nécrose des tumeurs tels que TNF-α et TNF-β, facteurs de croissance des fibroblastes (FGF), facteurs de croissance dérivés des plaquettes (PDGF), facteurs de croissance des globules blancs tels que G-CSF, GM-CSF, M-LSF, facteurs de croissance transformants tels que TGF-β.

8. Procédé selon la revendication 7, dans lequel les cytokines sont TNF-α, IL-12, interféron-α, FGF, et PDGF.

9. Procédé selon la revendication 5 ou la revendication 6, dans lequel les chimiokines comprennent des chimiokines CC et CXC.

10. Procédé selon l'une quelconque des revendications précédentes, dans lequel le procédé comprend en outre l'étape supplémentaire de détermination de la concentration de protéine 1 chimioattractante pour les macrophages (MCP-1) dans un échantillon du même patient.

11. Procédé selon l'une quelconque des revendications précédentes, dans lequel le procédé comprend en outre l'étape supplémentaire de détermination des changements statistiques dans des paramètres immunologiques.

12. Procédé selon la revendication 11, dans lequel le paramètre immunologique est les rapports lymphocytaires.

13. Procédé selon l'une quelconque des revendications précédentes, dans lequel le procédé comprend en outre l'étape d'évaluation de l'épaisseur de l'intima vasculaire pour déterminer s'il existe un épaississement qui pourrait être indicatif de la présence ou de la probabilité d'athérosclérose ou de plaques d'athérosclérose.

14. Procédé selon la revendication 13, dans lequel l'évaluation de l'épaisseur de l'intima vasculaire est par échographie.

15. Procédé selon l'une quelconque des revendications précédentes, dans lequel les données sont interprétées à l'aide d'un logiciel informatique et de représentation graphique.

16. Procédé selon la revendication 3, dans lequel l'analyse de l'échantillon de sang comprend le marquage du sang total dans un tube unique.

17. Procédé selon la revendication 16, dans lequel l'analyse de l'échantillon de sang comprend les étapes supplémentaires de lyse des globules rouges, et la lecture à l'aide d'une cytométrie de flux.

18. Procédé selon la revendication 16, dans lequel l'analyse de l'échantillon de sang comprend les étapes supplémentaires d'addition de cellules marquées à une phase solide, et l'utilisation d'un analyseur d'images pour compter les cellules.

19. Procédé selon la revendication 17 ou la revendication 18, dans lequel le logiciel d'ordinateur est utilisé pour comparer des résultats de patients uniques à une base de données de résultats d'individus en bonne santé et à une base de données de données de différentes étapes de la maladie pour faciliter la prédiction précise de la formation de plaques.
